# EUROPEAN PATENT APPLICATION

(11) **EP 3 718 549 A1**
(43) Date of publication of application: **07.10.2020**
(21) Application number: 18883309.9
(22) Date of filing: 01.11.2018
(51) Int. Cl.: A61K 31/5513, A61K 31/5517, A61K 31/15, G01N 33/50, G01N 33/94

(54) **NOVEL PHARMACEUTICAL COMPOSITION FOR TREATMENT OF DYSTONIA**

(30) Priority: 28.11.2017 KR 20170160506
(71) Applicant: Korea Advanced Institute of Science and Technology, Daejeon 34141 (KR)
(72) Inventor: KIM, Dae Soo, Daejeon 34141 (KR); KIM, Jung Eun, Daejeon 34141 (KR)
(74) Representative: Dehns
(86) International application number: PCT/KR2018/013159
(87) International publication number: WO 2019/107767

(57) **Abstract**

The present invention relates to a novel pharmaceutical composition for the treatment of dystonia or relieving pain caused by myotonic condition in a subject suffering from dystonia. Particularly, the present invention provides a pharmaceutical composition for treating dystonia or relieving pain caused by dystonia comprising a serotonin receptor 5-HT_{2A} inhibitor as an active ingredient.

## Description

### Technical Field

The present application claims priority to Korean Patent Application No. 2017-0160506 filed on November 28, 2017. The contents of the above patent application are incorporated by reference into this document as a whole.

The present invention relates to a novel pharmaceutical composition, and more particularly, to a novel pharmaceutical composition for treating dystonia.

### Background Art

Dystonia is a symptom which is characterized by an involuntary abnormal movement phenomenon that causes an abnormal posture or twisting muscle movement regardless of one's will. It is a neurological disease. These symptoms involuntarily cause the muscles to contract, resulting in abnormal movements and strange postures, such as muscle twisting or repetitive movements. Dystonia can occur as a genetic cause or as a secondary sign of a specific cause disease. However, in relation to the pathogenesis of dystonia, symptoms similar to dystonia appear when a GABA antagonist such as bicuculline is injected into an animal, and thus, it is only assumed that dystonia develops when a problem occurs in the neurotransmitter GABA-related neural network (Inase et al., J. Neurophysiol. 75: 1087-1104, 1996), but the exact mechanism of the onset is not clearly known.

In addition, currently, administration of Botox® is in use clinically, and a treatment that has a lasting effect of about 3 months. However, the treatment using Botox® is complex and expensive.

On the other hand, as a treatment method for dystonia, an electric stimulation treatment apparatus has been proposed to apply local electric stimulation to a muscle region on where dystonia symptoms appear (US20160296756A1). However, the prior art has little effect on muscle tension caused by stress factors.

### Summary of the Invention

The present invention is derived to solve a number of problems, including the above problems, and more particularly the present invention provides a novel pharmaceutical composition for treating dystonia, in particular, treatment of dystonia due to stress and relief of pain caused by dystonia. However, the scope of the present invention is not limited thereto.

In an aspect of the present invention, the provided is a pharmaceutical composition for treating dystonia or relieving pain caused by myotonic condition comprising the serotonin receptor 5-HT_{2A} inhibitor as an active ingredient.

In another aspect of the present invention, the provided is a method of screening candidate of therapeutic substance for treating dystonia or relieving pain caused by dystonia, comprising:
observing whether a series of test compounds or natural products inhibits serotonin receptor 5-HT_{2A}; and
selecting a test compound or a natural product identified as inhibiting the 5-HT_{2A}.

In another aspect of the present invention, the provided is a method for treating dystonia in a subject having symptoms of dystonia caused by stress, comprising administering therapeutically effective amount of a serotonin receptor 5-HT_{2A} inhibitor to the subject.

In another aspect of the present invention, the provided is a method for alleviating pain caused by dystonia in a subject having symptom of dystonia caused by stress, comprising administering therapeutically effective amount of a serotonin receptor 5-HT_{2A} inhibitor to the subject.

In another aspect of the present invention, the provided is a method for preventing dystonia in a subject concerned with dystonia due to stress, comprising administering a therapeutically effective amount of a serotonin receptor 5-HT_{2A} inhibitor to the subject.

In another aspect of the present invention, the provided is the use of a serotonin receptor 5-HT_{2A} inhibitor in the manufacture of a pharmaceutics for treating of dystonia or relieving pain caused by dystonia.

### Effect of the Invention

As described above, according to an embodiment of the present invention, dystonia caused by not only genetic factor, but also extreme stress can be effectively treated and prevented. Of course, the scope of the present invention is not limited to these effects.

### Brief Description of Drawings

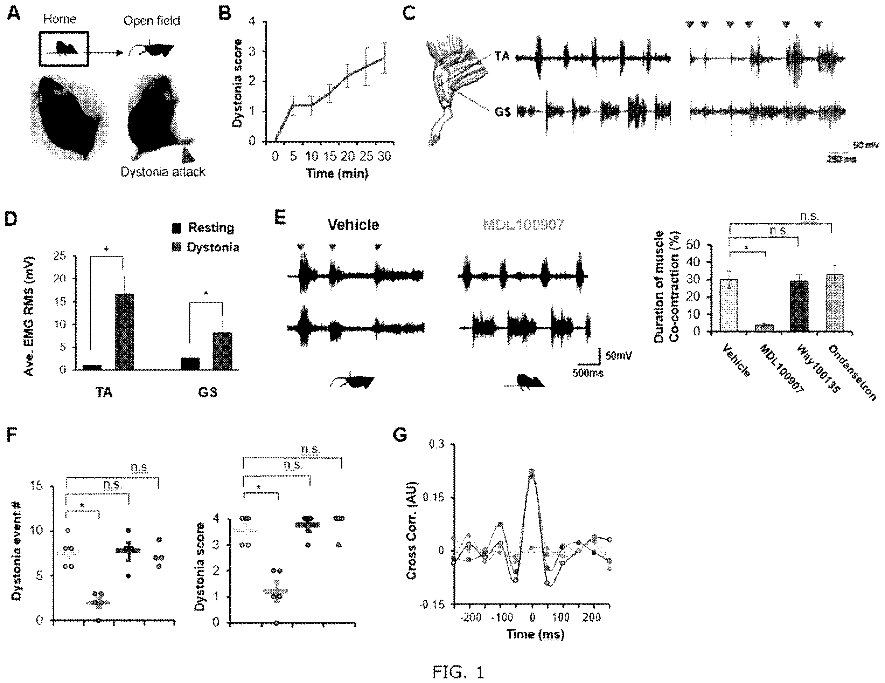

FIG. 1 represents the results of observing symptoms of dystonia in dystonia model animals by various analysis methods according to an embodiment of the present invention. (A) Overview of the animal experiment according to an embodiment of the present invention (top) and a picture of the hyperextension of the hind limbs of the experimental animal in dystonic posture (bottom); (B) a graph recording the change of dystonia score of tottering mice placed in the open field box over time of exposure of environmental stress; (C) electromyograms (EMG) measured in the extensor (gastrocnemius) and tibialis anterior muscle in normal state and dystonia, respectively; (D) comparison of mean RMS (reflecting muscle contraction or muscle tension) of extensor and tibialis anterior muscle in dystonia and resting state; (E) a series of graphs representing the rate of co-activation in two muscles (gastrocnemius and tibialis anterior muscle) after administrating vehicle (control) and three types of serotonin receptor antagonists (MDL100907, Way100135 and Ondansetron) to dystonia model animals; (F) a series of graphs representing number of dystonia events (left) and dystonia score (right) after administrating various serotonin receptor antagonists; (G) a graph representing the cross-correlation after administrating various different serotonin receptor antagonists.

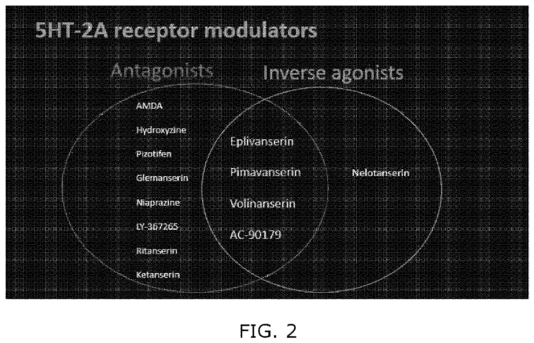

FIG. 2 is a Venn-diagram representing each of an "antagonist" which inhibits activation of a receptor by interfering with agonist binding thereto but does not affect activation/inactivation equilibrium and an "inverse agonist", a drug that stabilizes a target molecule with an inactive structure.

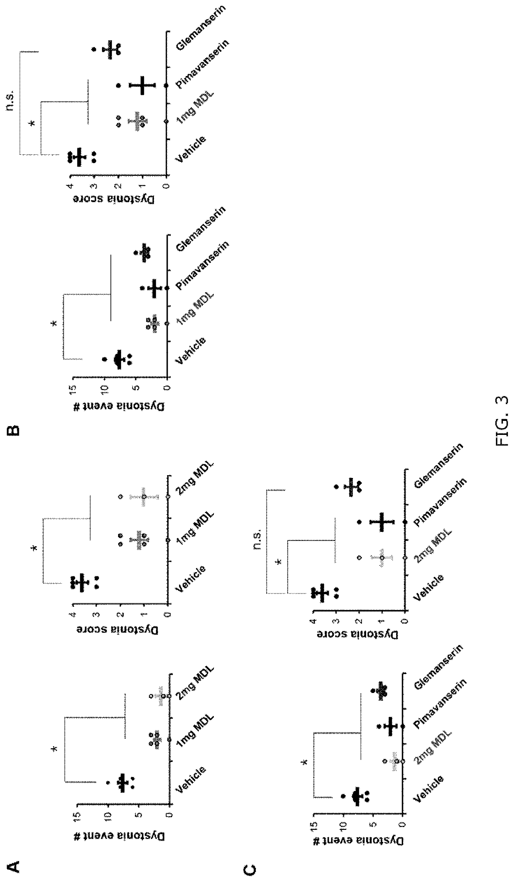

FIG. 3 represents the results of measuring the symptoms of dystonia in dystonia model animals according to the administration of various serotonin receptor 5-HT_{2A} antagonists. (A) A series of graphs showing the results of measuring the number of dystonia events (left) and dystonia score (right) after administrating MDL100907 to dystonia model animals dose dependently, (B) a series of graphs showing the results of measuring the numbers of dystonia events (left) and dystonia score (right) after administrating vehicle (control) and three types of serotonin receptor 5-HT_{2A} antagonists (1mg MDL100907, Pimavanserin and Glemanserin) to dystonia model animals; and (C) a graph in which only the concentration of one group was changed to 2 mg of MDL100907 in B.

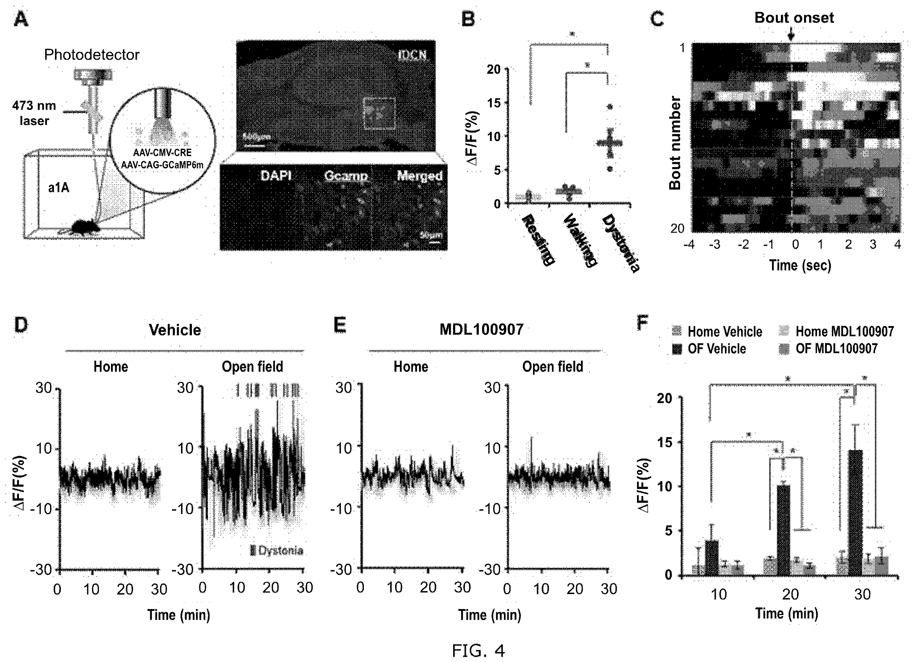

FIG. 4 represents the results of real-time fluorescence analysis in the cerebellum of GCaMP6 topical transduced mice, according to one embodiment of the present invention. (A) Overview of the real-time fluorescence analysis system used in the present invention (left), fluorescence microscopy photograph observing fluorescence from cerebellar sections extracted from experimental animals (right); (B) a graph showing the rate of fluorescence change according to the behavioral state of experimental animals; (C) hit map of aligned fluorescence changes of starting sections related to dystonia in the Vehicle group; (D, E) a series of graphs showing the rate of fluorescence change of control group and MDL100907 administration group when in home and after moving to the open field box; respectively; and (F) a graph showing the rate of fluorescence change in the control group, and a drug administration group, when in home and after moving to the open field box according to exposure time to the environmental stress.

### Best Modes

### Definition of Terms:

The term "dystonia" as used in this document is an involuntary abnormal movement that leads to persistent postural or twisting muscle movements irrespective of one's will. As a neurological disease characterized by symptoms, familial or stress-related dystonia is known to exist. In particular, the stress-related dystonia may be caused by extreme stress ahead of a performance or presentation, even if there is no other genetic predisposition.

The term "cerebral palsy" as used in this document refers to a state in which motor function is paralyzed due to damage to the brain, and is known to be caused by brain damage at birth, symptomatic jaundice in newborns, and meningitis. The most important symptom is that the neuromuscular system is not properly controlled, and about 70 to 80% of patients with cerebral palsy are known to have spastic cerebral palsy, which causes muscles to become rigid and difficult to move, causing abnormal muscle tone.

The term "myotonic dsytrophy," as used in this document, is also called Steinert's disease and is a disease in which muscles are gradually weakened. When symptoms occur in various places in the body, they appear in various types. Symptoms are muscle stiffness. In general, muscle relaxation comes after muscle contraction. In patients with this disease, muscle relaxation progresses slowly and there is stiffness in the overall muscle.

The term "spinobulbar muscular atrophy", as used in this document, is also called Kennedy's disease and is a sex-linked inherited disease caused by mutation of the androgen receptor gene on the X chromosome. When repeated duplication of the gene is occurred, the function of androgen receptor does not work well resulting in regression of motor neurons. Symptoms do not appear before age 30, but after 10-20 years, muscle disorders such as muscle stiffness begin in earnest, requiring wheelchairs, and impediments in talking or swallowing occur.

The term "serotonin receptor" used in this document refers to a receptor that binds to neurotransmitter serotonin (5-HT) and causes physiological activity in cells. In mammals, it is present in high concentrations in the peripheral nervous system, and brain nuclei, substance nigra, globus pallidus, basal ganglia, and choroid plexus in the brain, and they are known to regulate K⁺ or Ca²⁺ ion channels via G proteins or cAMP as secondary signaling molecules. li is kwon that serotonin receptors include 14 subtypes of 5-HT_{1A}, 5-HT_{1B}, 5-HT_{1D}, 5-HT_{1E}. 5-HT_{1F}, 5-HT_{2A}, 5-HT_{2B}, 5-HT_{2C}, 5-HT₃, 5-HT₄, 5-HT_{5A}, 5-HT_{5B}, 5-HT₆, and 5-HT₇ exist, and these subtype. It is known that the function, antagonist, and agonist etc. differ slightly depending these subtypes.

As used herein, the term "antagonist" refers to a ligand or drug that serves to block or attenuate a biological reaction by binding to the target molecule and blocking its function, rather than activating the target molecule, such as an agonist.

As used herein, the term "selective antagonist" refers to a ligand or drug that selectively blocks the function of only a specific subtype in a target molecule having various subtypes.

As used herein, the term "dual antagonist" refers to an antagonist that inhibits two or more subtypes that are structurally closely related to a target molecule having various subtypes. For example, the 5-HT_{2A} and 5-HT_{2C} used in the present invention are structurally very close receptors and there are antagonists inhibiting only these two subtypes by distinguishing from the other subtypes but not distinguishing the difference between these two subtypes. However, they have the advantage of having fewer side effects than non-discriminatory non-selective antagonists, although they have more side effects than selective antagonists.

The term "inverse agonist" as used herein refers to a drug that stabilizes a target molecule to an inactive structure. In general, a target molecule such as a receptor has an activated structure and an inactivated structure in a balanced state, and the equilibrium in the activated structure is inclined dependent on agonists. Antagonists interfere with activation by interfering with agonist binding, but do not affect activation/deactivation equilibrium. The inverse agonist acts by inclining the equilibrium state to the inactivation structure, and thus can inhibit the activity of the receptor permanently.

As used herein, the term "antagonistic antibody" refers to an antibody that binds to a specific target protein and inhibits the physiological activity of the target protein.

The term "functional fragment of an antibody" as used herein refers to a fragment of an antibody such as Fab, Fab', F(ab')₂ which has a conserved antigen-binding site produced by cleaving the antibody with a protein cleavage enzyme, or scFv, diabody, tribody, which are single chain-based antibody analogues prepared by linking antibody heavy chain variable region (V_{H}) and antibody light chain variable region (V_{L}) by a linker or more comprehensively, other single chain-based antibody analogues including sdAb, V_{H}H, nanobody, monobody, variable lymphocyte receptor (VLR), Affilin, Affimer, Affitin, Avimer, DARPin, Fynomer, Affibody.

The term "Fab" used in this document is a fragment antigen-binding, a fragment produced by cutting an antibody molecule with a protease, papain, a heterodimer consisting of two peptides, V_{H}-CH1 and V_{L}-C_{L}. and another fragment produced by papain is called a fragment crystalizable (Fc).

The term "F(ab')₂" used in this document is a fragment containing an antigen-binding site among fragments produced by cleaving an antibody with a protease, pepsin, and refers to a tetrameric form in which the two Fabs are connected by disulfide bonds. Another fragment produced by pepsin is referred to as pFc'.

The term "Fab"' used in this document is an antibody fragment having a similar resemblance to the Fab, and is produced by reducing the F(ab')₂, and the length of the heavy chain portion is slightly longer than that of Fab.

The term "scFv" used in this document refers to a single chain variable region fragment which is a recombinant antibody fragment prepared as a single chain by linking variable regions (V_{H} and V_{L}) among Fab of the antibody with a linker peptide as a single chain.

The term "sdAb (single domain antibody)" used in this document is referred to as a nanobody which is an antibody fragment consisting of a single monomeric variable region fragment of an antibody. The sdAb derived from the heavy chain is mainly used, but a single variable region fragment derived from the light chain is also reported to be a specific binding to the antigen.

The term "V_{H}H" used in this document is a variable region fragment of the heavy chain of IgG composed only of dimers of heavy chains found in camels, and has the smallest size (∼ 15 kD) among antibody fragments that specifically bind antigen. It was developed by Ablynix under the brand name Nanobodies®.

As used herein, "Fv (fragment variable)" is a dimeric antibody fragment consisting only of variable and variable chains (V_{H} and V_{L}) of the heavy and light chains of the antibody, the size of which is between sdAb and Fab (about ∼ 25 kD). Is produced by protein hydrolysis under special conditions or by inserting and expressing V_{H} and V_{L}-encoding genes into one expression vector.

As used herein, "diabody" is a divalent bispecific recombinant antibody fragment prepare by shortening the length of the linker between V_{H} and V_{L} of the scFv (5 a.a.) so that two scFvs form dimers with each other which is known to have a higher antigen specificity than conventional scFv.

The term "calcium sensor protein" used in this document is a fluorescent protein genetically engineered to measure changes in the concentration of calcium ions in a cell by fluorescence change, and is also called a fluorescent Ca²⁺ indicator protein (FCIP). It is mainly a fusion protein in which a calcium ion-binding protein such as calmodulin is linked to a fluorescent protein such as GFP by a linker. It is used for detecting intracellular calcium ion concentration by detecting fluorescence emitted only when calcium ion is bound by structural changes directly, or by using fluorescence resonance energy transfer (FRET) phenomena between two fluorescent proteins according to a structural change when binding with calcium ion.

### Detailed Description of the Invention

In an aspect of the present invention, the provided is a pharmaceutical composition for treating dystonia or relieving pain caused by myotonic condition comprising the serotonin receptor 5-HT_{2A} inhibitor as an active ingredient.

As used herein, the term 'treating' is a concept that includes the elimination of pathological causes and the improvement or relief of symptoms.

In the composition, the myotonic condition may be caused by dystonia, cerebral palsy, myotonic dystrophy or spinobulbar muscular atrophy. Myotonic symptoms include not only dystonia, but also various neurological disorders, particularly cerebral palsy (Asagai et al., Laser Therapy, 14 (4): 171-178, 2005), myotonia dystrophy (Wenninger et al., Front. Neurol. 9: 303, 2018), and spinal cord muscle atrophy (Araki et al., Neuromuscul. Disord. 25 (11): 913-915, 2015). The myotonic condition may be due to excessive stress, an abnormal increase in serotonin following administration of a selective serotonin reuptake inhibitor, or abnormal activation of the serotonin cycle. In particular, the myotonia may be caused by side effects of administration of a selective serotonin reuptake inhibitor ('SSRI'), which is an antidepressant. It is a drug that treats depression by increasing the concentration of serotonin at the extracellular level by inhibiting reuptake into cells (presynaptic cells). However, in some patients, as a side effect of SSRI administration, there is a problem of having muscle tension due to excessive increase of serotonin at the synapse, and for this reason, depression patients suffering from muscle tension frequently stop taking SSRI (Mossavi et. al., Glob. J. Health Sci. 6(6): 295-299, 2014). Therefore, the pharmaceutical composition of the present invention can be used for the purpose of preventing or alleviating dystonia symptoms of depression patients prescribed SSRIs. In this case, the selective serotonin reuptake inhibitors include citalopram, dapoxetine, escitalopram, fluvoxamine, paroxetine, fluoxetine, and fluoexetin. It can be traline (sertraline), zimeldin, or vortioxetine.

In the pharmaceutical composition, the 5-HT_{2A} inhibitor may be a 5-HT_{2A} antagonist or a 5-HT_{2A} inverse agonist, and the 5-HT_{2A} antagonist specifically binds the 5-HT_{2A}. The 5-HT_{2A} antagonist may be an antagonistic antibody or a functional fragment of the antagonizing antibody or clozapine, olanazapin, qutiapine, risperidone, ziprasidone, aripiprazole, acenapine, amitriptyline, clomipramine, amitriptyline, clomipramine, cyproheptadine, eplivanserin, etoperidon, haloperidol, hydroxyzine, iloperidone, ketanserin, methysergide, mianserin, mirtazapine, nefazodone, pimavanserin, pizotifen, ritanserin, trazodone or yohimbine. Alternatively, the 5-HT_{2A} antagonist may be a selective antagonist which does not act on other types of serotonin receptors or a 5-HT_{2A}/_{2C} dual antagonist. The 5-HT_{2A} selective antagonist may be eplivanserin, 2-alkyl-4-aryl-tetrahydro-pyramido-azepine, AMDA (9-aminomethyl-9,10-dihydro-anthracene), hydroxyzine, pizotifen, 5-methoxy-N-(4-bromobenzyl) tryptamine (5-MeO-NBpBrT), glemanserin, niaprazine, pimavanserin, volinanserin, or LY-367265, and the 5-HT_{2A}/_{2C} dual antagonist may be ritanserin, ketanserin, cyproheptadine, AC-90179, trazodone or etoperidone.

Meanwhile, the 5-HT_{2A} inverse agonist may be AC-90179, pimavanserin, nelotanserin, volinanserin, or eplivanserin.

The functional fragment of the antagonizing antibody may be Fab, Fab', F(ab')₂, scFv, diabody, tribody, sdAb, V_{H}H, nanobody, monobody, variable lymphocyte receptor (VLR), Affilin, Affimer, Affitin, Avimer, DARPin, Fynomer, or Affibody.

The pharmaceutical composition of the present invention may include at least one pharmaceutically acceptable carrier. The pharmaceutically acceptable carrier may be a variety of oral or parenteral formulations but is preferably a parenteral formulation. In the case of formulation, it is prepared using diluents or excipients such as fillers, extenders, binders, wetting agents, disintegrating agents and surfactants. Solid preparations for oral administration include tablets, pills, powders, granules, capsules, etc. These solid preparations may be prepared by mixing at least one compound and at least one excipient such as starch, calcium carbonate, sucrose or lactose, gelatin, etc. It is prepared by mixing. In addition, lubricants such as magnesium stearate and talc may be used in addition to simple excipients. Liquid preparations for oral administration include suspending agents, oral liquids, emulsions, syrups, etc. and these liquid preparations may include other various excipients such as wetting agents, sweeteners, fragrances, and preservatives besides water and liquid paraffin, which are commonly used as diluents. Formulations for parenteral administration include sterile aqueous solutions, non-aqueous solvents, suspensions, emulsions, lyophilized preparations, and suppositories. As the non-aqueous solvent and suspensions, propylene glycol, polyethylene glycol, vegetable oil such as olive oil, and injectable ester such as ethyl oleate may be used. As a base for suppositories, witepsol, macrogol, tween 61, cacao oil, laurin oil, and glycerogelatin etc. may be used.

The pharmaceutical composition of the present invention may be any one preparation selected from the group consisting of tablets, pills, powders, granules, capsules, suspensions, solutions, emulsions, syrups, sterilized aqueous solutions, non-aqueous solvents, suspensions, emulsions, lyophilizers and suppositories.

The pharmaceutical composition of the present invention may be administered orally or parenterally, and when administered parenterally, it is possible to administer through various routes such as intravenous injection, intranasal inhalation, intramuscular administration, intraperitoneal administration, and percutaneous absorption.

The pharmaceutical composition of the present invention may be administered in a pharmaceutically effective amount.

The term "pharmaceutically effective amount" as used herein refers to an amount sufficient to treat the disease at a reasonable benefit/risk ratio applicable to medical treatment, and effective dosage may be determined according to factors including the type, severity, age, sex, drug activity, drug sensitivity of subjects, time of administration time, route of administration, rete of excretion, duration of treatment, concurrently used drugs, and other factors well known in the medical field. The pharmaceutical composition of the present invention may be administered at a dose of 0.1 mg/kg to 1 g/kg, more preferably 1 mg/kg to 500 mg/kg. Meanwhile, the dosage may be appropriately adjusted according to the patient's age, gender and condition.

In another aspect of the present invention, the provided is a method of screening candidate of therapeutic substance for treating dystonia or relieving pain caused by dystonia, comprising:
observing whether a series of test compounds or natural products inhibits serotonin receptor 5-HT_{2A}; and
selecting test compounds or natural products identified as inhibiting the 5-HT_{2A}.

In another aspect of the present invention, the provided is a method for treating dystonia, comprising administering a serotonin receptor 5-HT_{2A} inhibitor to an individual having symptoms of dystonia caused by stress.

The method of screening may include determining whether the test compounds or natural products that have been confirmed to inhibit 5-HT_{2A} inhibits the function of the serotonin receptor except 5-HT_{2A}; and selecting test compounds or natural products that do not inhibit the function of the serotonin receptor except for 5-HT_{2A}.

Determining whether the test compounds or natural products that have been confirmed to inhibit 5-HT_{2A} inhibits the function of the serotonin receptor except 5-HT_{2A} may be performed through various analysis methods *in vitro, in vivo* or *in silico.* The *in vitro* method may use reacting the serotonin receptor 5-HT_{2A}, a ligand of the 5-HT_{2A} (e.g., serotonin) and the test compounds or natural products, and selecting test compounds or natural products that inhibit the binding of the serotonin 5-HT_{2A} and the ligand. The determining whether the test compounds or natural products inhibit the binding of the serotonin receptor 5-HT_{2A} and the ligand may be performed through various methods for analyzing the interaction between various target proteins and their ligands, which include radioisotope-labeled cell-compound-compact chromatography, drugs affinity responsive target stability (DARTS), stability of proteins from rate of oxidation (SPROX), differential static light scattering DSLS analysis, differential scanning fluorescence measurement (DSF), and differential radial capillary action of ligand assay (DraCALA), and such analysis methods has been introduced in detail by McFedries et al. (Chem. Biol., 20: 667-673, 2013). The above document is incorporated herein by reference.

Alternatively, the *in vitro* method may include analyzing the concentration or activity of the signal molecule downstream of 5-HT_{2A} after treating the test compound or the natural product with cells expressing the serotonin receptor 5-HT_{2A}; and selecting a test compounds or natural products that inhibit the concentration or activity of the signal molecule downstream of 5-HT_{2A}.

The signal molecule may be inositol triphosphate (IP₃), diacylglycerol (DAG), arachidonic acid (AA), 2-arachidonylglycerol (2-AG), Ca²⁺ or PKC.

In the screening method, the *in vivo* method includes inducing stress in the tottering animals having a mutation in P/Q type calcium channel by leaving the animals in a stress condition; administering a test compound or a natural product to the tottering animals under stress; and selecting a test compound or a natural product which significantly reduced the dystonia score of the tottering animals.

The term "stress" as used in this document refers to the psychological and physical tension that a subject feels when exposed to an environment that is difficult to adapt. Accordingly, the "stress condition" refers to a state in which the subject is exposed to an unfamiliar and unfamiliar environment. These stressful conditions are when a person encounters a new environment, such as contacting new people, presenting in a new place, taking on new tasks, moving to a new area, or going to a higher school. If one develops dystonia under stress conditions, he/she will have a major obstacle in his/her social life.

Optionally, the *in vivo* method may comprise transducing a gene encoding calcium sensor protein topically into the cerebellum of the a tottering animal having a mutation in P/Q type calcium channel in addition to the measurement of the dystonia score of the tottering animal; inducing stress in the tottering animal by leaving the animal in a stress condition; administering the test compound or natural product to the tottering animal under stress; measuring the amount of calcium sensor protein bound to calcium in the cerebellum of the tottering animal; and selecting a test compound or natural product that significantly lowers the amount of calcium sensor protein bound to calcium.

In the screening method, the calcium sensor protein is yellow cameleon (YC), Inverse-Pericam (Nagai et al., Proc. Natl. Acad. Sci. USA. 98(6): 3197-3202, 2001), Camgroo (Baird et al., Proc. Natl. Acad. Sci. USA., 96 (20): 11241-11246, 1999; Griesbeck et al., J. Biol. Chem. 276(31): 29188-29194, 2001), TN-L15 (Heim and Griesbeck, J. Biol. Chem., 279(14): 14280-14286, 2004), SynapCam (Guerrero et al., Nat. Neurosci., 8(9): 1188-1196, 2005) or GCaMP. The YC may be YC2.1, YC 3.1, YC 2.12, YC 3.12, or YC 3.60, the Camgroo may be Camgroo-1, or Camgroo-2, and the GCaMP may be GCaMP1, GCaMP2, GCaMP3, GCaMP4, GCaMP5 or GCaMP6. Among these, GCaMP is a calcium sensor first developed by Junichi Nakai. It is a fusion protein composed of cpEGFP, calmodulin and M13 which is a peptide sequence from myosin light chain kinase and has a characteristic of exhibiting fluorescence in proportion to intracellular calcium ion level. It can be used to measure intracellular calcium ion level, and it can be particularly useful for measuring the concentration of calcium ions in specific tissues or cells in living cells or animals in real time (Nakai et al., Nat. Biotehonol., 19: 137-141, 2001).

In another aspect of the present invention, the provided is a method for treating dystonia in a subject having symptoms of dystonia caused by stress, comprising administering therapeutically effective amount of a serotonin receptor 5-HT_{2A} inhibitor to the subject.

In the method, the serotonin receptor 5-HT_{2A} inhibitor is as described above.

In another aspect of the present invention, the provided is a method for alleviating pain caused by dystonia in a subject having symptom of dystonia caused by stress, comprising administering therapeutically effective amount of a serotonin receptor 5-HT_{2A} inhibitor to the subject.

In the method, the serotonin receptor 5-HT_{2A} inhibitor is as described above.

In another aspect of the present invention, the provided is a method for preventing dystonia in a subject concerned with dystonia due to stress, comprising administering a therapeutically effective amount of a serotonin receptor 5-HT_{2A} inhibitor to the subject.

In the method, the serotonin receptor 5-HT_{2A} inhibitor is as described above.

In another aspect of the present invention, the provided is the use of a serotonin receptor 5-HT_{2A} inhibitor in the manufacture of a pharmaceutical agent for treating dystonia or relieving pain caused by dystonia.

In the use, the serotonin receptor 5-HT_{2A} inhibitor is as described above.

The present inventors use the tottering mouse prepared by inducing a mutation in the P/Q type calcium channel, which causes the dystonia symptoms to worsen with the severity of muscle tension when constant stress is applied to the experimental animals. As a result of measuring dystonia score and electromyogram (EMG), it was confirmed that the dystonia symptoms worsened over time, and when selective antagonists of 5-HT_{1A}, 5-HT_{2A} and 5-HT₃ were administrated to the experimental animals it was confirmed that only selective 5-HT_{2A} antagonist can alleviate dystonia symptoms (see FIG. 1). In addition, the effects of the drugs were screened by comparing the degree of relief of dystonia according to the binding affinity of selective 5-HT_{2A} antagonists to 5-HT_{2A} receptor (see FIG. 2 and Table 1). Specifically, Pimavanserin and MDL100907, which can be used simultaneously as antagonists and inverse agonists significantly, reduced symptoms of dystonia compared to Glemanserin, which acts only as an antagonist (see FIG. 3).

**Table 1**

| Several properties of major selective 5-HT_{2A} antagonists | | | |
|---|---|---|---|
| Drugs | Ki (nM) | pKi | IC₅₀ (nM) |
| Pimavanserin | 0.1995 | 9.7 | pIC₅₀=8.73 |
| Nelotanserin | 0.4 ± 0.07 | | |
| Ketanserin | 0.49/1.25 | 8.8 | |
| LY-367265 | 0.81 | | |
| Volinanserin (MDL100907) | 0.85 | 6.6 | |
| Ritanserin | 1.58 | 8.8 | |
| AC-90179 | 2.1 ± 0.2 | | |
| Glemanserin | 2.5 | | 6.5 ± 2 |
| Pizotifen | 7.5 | | |
| AMDA | 20 | | |
| Niaprazine | 75 | 7.61 ± 0.18 | |
| Eplivanserin | Sleep pill (approval was withdrawn) | | PIC₅₀=1.30 |
| Hydroxyzine | Histamine receptor antagonist | | 170 |

In addition, in view of the fact that 5-HT_{2A} is involved in the release of calcium into cells, the present inventors measured the concentration of calcium ions in the cerebellum of stress-induced dystonia model animals using the GCaMP6 method. As a result, the calcium ion concentration in the cerebellum was significantly increased in animals directly displaying dystonia symptoms, but it was confirmed that the increase in calcium ion concentration in the cerebellum was suppressed in the group administered with the 5-HT_{2A} selective antagonist (see FIG. 4).

As can be seen through the above-described experimental results, the pharmaceutical composition comprising a selective 5-HT_{2A} antagonist according to an embodiment of the present invention can be used effectively for the patients suffering from dystonia, especially for dystonia patients caused by stress conditions. In addition, an effective option with respect to the prevention of outbreaks of dystonia is to prescribe patients the pharmaceutical composition of the present invention before stressful situations. It can prevent symptoms of dystonia and improve patients' quality of life. In addition, it is possible to suppress mistakes caused by abnormal muscle tension by lowering the effect of muscle tension in presenters preparing to present, players preparing to perform and athletes ahead of the game in front of many people as well as dystonia patients. Normally, in a tense situation, a slight muscle tension is transferred to a mental burden, which in turn develops into a large muscle tension, which is thought to be effectively treated or preventable by the pharmaceutical composition of the present invention. It is usually thought to be useful for voice tremors or muscle tension when one performs a presentation. Also, it is thought that it is possible to prevent and treat muscle dysfunction caused by excessive muscle tension by ingesting the pharmaceutical composition of the present invention before and after muscle tension, not only in dystonia patients but also in normal people at an appropriate concentration.

In addition, it is believed that the discovery of therapeutic drugs will be effective not only for dystonia, but also for exercise disorders such as Parkinson's disease and tick disorders exacerbated by stress.

In addition, the experimental animal model used in the present invention can be used to confirm the medicinal effect of a candidate therapeutic agent by observing whether muscle tension is relieved through muscle electromyography analysis, and furthermore, it displays calcium-specific fluorescence in brain neurons. Calcium sensor protein can be useful for screening candidate drugs for dystonia by quantitatively confirming the effect of a candidate drug by changing the concentration of calcium ion in a specific brain region while a living animal is acting.

In addition, until now, there is a lack of reliable associated biomarkers for dystonia, so there is no way to distinguish various disease mechanisms in the patient group. This may reduce the effectiveness of the treatment or contribute to a false diagnosis. Thus, the present invention provides a method for confirming the activity of the 5-HT_{2A} receptor in the parietal nucleus region through brain imaging techniques (MRI, PET) as an associated biomarker for screening a patient group with dystonia. This is a method of regulating the amount of serotonin using a drug and confirming the activity of the receptor with a PET image using [¹¹C] NMSP, a 5-HT_{2A} receptor specific radioligand (Nordstrom AL et al., Int. J. Neuropsychopharmacol. 11 (2): 163-171, 2008; Kornum BR et al., J. Cereb. Blood Flow Metab., 29 (1): 186-196, 2009). To regulate the amount of serotonin, escitalopram, citalopram, or DOI which is an agonist of 5-HT_{2A} receptors can be used. Through this, it is possible to specifically select patients called "Task specific dystonia" who develop symptoms when starting a specific activity in the patient group and can provide effective dosing conditions and methods of treatment.

### Mode for the Invention

Hereinafter, the present invention will be described in more detail through following examples. However, the present invention is not limited to the examples disclosed below, but can be implemented in various different forms, and the following examples make the disclosure of the present invention complete, and inform the full scope of the invention to those skilled in the art completely.

### Example 1: Preparation of experimental animals

The 7-8 weeks old CACNA1A*tot*/*tot* mice (Fletcher, Cell, 87 (4): 607-617, 1996, The Jackson Laboratory, Stock No: 000544) were selected kept and dealt complied with the regulation (protocol number KA2015-05) of Institutional Animal Care and Use Committee of the Korea Advanced Institute of Science and Technology (KAIST). The mice remained freely accessible to water and feed, and the light-dark cycle was 12/12 hours. Behavioral tests were performed 4 weeks after the viral transduction. In the CACNA1A^{*tot*/*tot*} mice, the proline present in the repeat region II S5-S6 of Cav2.1, which is a P/Q-type calcium channel, is replaced with a leucine residue, the activity of the P/Q-type channel is reduced in neurons thereby. According to previous studies, it has been reported that the expression of Cav1.2, which is an L-type calcium channel, is increased according to a decrease in P/Q-type channel activity (Fletcher, Cell, 87(4): 607-617, 1996), It has been reported that when the tottering mice are placed in a new environment, dystonia occurs after about 10 minutes (Alvina, K., & Khodakhah, K., Neurosci., 30(21): 7258-7268, 2010).

### Example 2: Construction of topical transgenic tottering mice

To analyze the correlation between the degree of dystonia symptoms and the concentration of calcium ions in neurons, optogenetic model animals were prepared. Particularly, for a photometric system with a polarization-maintaining single-mode optical fiber, AAV2/9-CAG-FLEX-GCaMP6m (Izadmehr et al., Cell, 164: 617-631, 2016) and AAV2/9-CMV-Cre (Gompf et al., Front. Behav. Neurosci. 9: 152, 2015) viral vectors were injected unilaterally into 0.25 µl each of the fastigial nucleus of the CACNA1A*tot*/*tot* mouse through stereotaxic surgery. The virus injection was carried out 4 weeks before the experiment. Subsequently, the optical fiber inserted in the stainless-steel heat-resistant conduit was implanted directly above the injection site. For the fluorescence measurement, a TCSPC-based light measurement system (Becker & Hickel, Germany) was used.

### Experimental Example 1: Confirmation of changes in dystonia symptoms according to administration of various serotonin receptor antagonists

A selective 5-HT_{1A} antagonist Way100135, a selective 5-HT_{2A} antagonist MDL100907 (volinanserin) and a 5-TH₃ selective antagonist ondansetron were all purchased from Sigma Aldrich. The drugs were dissolved in physiological saline and administrated intravenously to the tottering animal prepared in the Example 2 and kept in the controlled environment of 12/12 hour light-dark cycle. As previously reported, the Way100135 (10 mg/kg, Loscher et al., Eur. J. Pharmacol., 255(1-3): 235-238, 1994), MDL100907 (1 mg/kg, Barr et al., Neuropsychopharmacol., 29(2): 221-228, 2004) or ondansetron (1 mg/kg, Minville et al., Br. J. Anaesth., 106 (1): 112-118, 2010) were administrated to the tottering mice, respectively, and for the control record, the tottering mice were intravenously injected with the same volume of physiological saline. The behavior test was performed 30 minutes after drug administration, and the experimental animals were acclimated in the home for 30 minutes and moved to an open field box, they were exposed to a stressed environment by leaving them in the open field box for 30 min, and the symptoms of dystonia were measured. The symptoms of dystonia and its severity were calculated using a 5-point scale dystonia score as follows: 0 = normal behavior, 1 = abnormal motor behavior, no myotonic posture, 2 = minor dyskinesia, showing dystonialike posture when disturbed, 3 = moderate motor disability, frequent spontaneous myotonic posture, 4 = severe motor disability, and persistent myotonic posture.

As a result, when the dystonia model animals were placed in a stressful situation, that is when the mice were exposed to an open-box box to measure dystonia symptoms over time, it was observed that tottering mice worsened symptoms over time. Particularly, it was observed that the symptoms of the dystonia model animals increased in proportion to the time exposed to the stressed environment. Especially, the symptoms were increased when 30 minutes elapsed compared to when 10 minutes elapsed.

In addition, as shown in FIG. 1F, when the three serotonin receptor antagonists were administered, respectively, it was confirmed that the selective 5-HT_{2A} antagonist MDL100907 (volinanserin) alleviated symptoms of dystonia significantly after measuring attack event number and muscle dysfunction index. However, the selective 5-HT_{1A} antagonist Way100135 and the selective 5-HT₃ antagonist ondansetron did not reduce symptoms of dystonia at all. This shows that dystonia symptoms are very limited to 5-HT_{2A}.

### Experimental Example 2: EMG measurement

Electrodes (A-M system, USA) were surgically inserted into the gastrocnemius (GS) and tibialis anterior (TA) muscle for EMG recording, respectively. The electrodes were made using multiple strands of stainless-steel wires attached to each other and coated with Teflon. After shaving the hair of the right hind leg and behind the neck, a small incision was formed in the skin of the dorsal neck area. Four sets of recording electrodes were connected from the neck incision to the muscles under the skin. The EMG wires were wired under the skin and connected to a connector fixed to the skull. The low impedance of the exposed end of the wires allows the capture of electrical signals that have passed through relatively long distances (Pearson et al., J. Neurosci. Methods, 148 (1): 36-42, 2005). The EMG signals from the electrodes were sampled at 10 kHz and digitized with MiniDigi 1A (Axon Instrument, USA). Raw traces were analyzed using Clampfit 9.2 (Axon Instrument, USA). The cross correlation was calculated by using the standard of NeuroExplorer (Ver. 4, Nex Technologies, USA). The cross correlation indicates that the dystonia symptoms are severe when a high dystonia score is indicated at 0 ms. The cross correlation indicates the degree of EMG overlap for a certain period in two muscles (TA and GS), and is a measure for judging whether the dystonia symptoms occur simultaneously in two muscles. This is a measure for the timing of muscle contraction between muscle parts, and the analysis method shows a higher value in the 0 ms portion of the X axis of time (ms) as the timings are similar to each other. That is, the greater the cross correlation value (Cross Corr.), which is the Y-axis value when the signal between each other is 0 ms (when contracted at the same time), the more severe the dystonia symptoms that the two muscles contract at the same time. The unit of the Y axis, AU, is a relative cross correlation value when the control is set to 1 which is an arbitrary unit.

As shown in FIG. 1C, in the case of the dystonia model animal used in the present invention, the muscle conduction in the tibialis anterior muscle (TA) and the gastrocnemius (GS) muscle was measured. When the dystonia model animals are exposed to a stress environment compared to a normal control group, it was exhibited a characteristic EMG pattern of dystonia.

Then, the present inventors administered the three serotonin receptor antagonists to dystonia model animals exposed to a stressed environment. As shown in FIG. 1G, the correlation disappeared upon administration of MDL100907, but there is no difference when Way100135, a selective 5-HT_{1A} antagonist, and Ondansetron, a selective 5-HT₃ antagonist are administrated, respectively, compared to the control group. This shows that the 5-HT_{2A} inhibitor according to an embodiment of the present invention can be very effective in alleviating dystonia symptoms, particularly stress-induced dystonia symptoms.

Then, the present inventors observed the dose-dependent effect of selective 5-HT_{2A} antagonist MDL100907 (volinanserin) on the tottering mice. As shown in FIG. 3A, it was confirmed that symptoms were slightly alleviated at 2 mg/kg (Padich, Robert A. et al., Psychopharmacol. 124(1-2): 107-116, 1996) rather than 1 mg/kg. In addition, the degree of relaxation of dystonia was measured when various serotonin 5-HT_{2A} receptor antagonists and inverse agonists were administered. As shown in FIGS. 3B and 3C, compared to the control group, the selective 5-HT_{2A} antagonist and inverse agonist MDL100907 and Pimavanserin (1.5 mg/kg, Goldman, JD, Parkinsons Dis., 675630, 2011) groups showed no dystonia paralysis. However, a 5-HT_{2A} antagonist Glemanserin (2 mg/kg, Harvey et al., Behav. Neurosci., 126 (4): 530, 2012) which is not an inverse agonist of 5-HT_{2A} showed moderate relief between the control and MDL100907 and Pimavanserin groups.

### Experimental Example 3: Measurement of calcium ion concentration change in vivo

From the above results, the present inventors focused on the fact that 5-HT_{2A} is closely related to calcium signaling in the cerebellum, and invested whether it is possible to diagnose the symptoms of dystonia and verify the therapeutic effect of selective 5-HT_{2A} antagonist by measuring the concentration of calcium ion in the cerebellum in the tottering mice. The change in calcium signaling in the cerebellum is possible by transduction of a gene encoding the calcium sensor protein GCaMP6 into the cerebellum of the dystonia model mouse, and then measuring the intensity of fluorescence of the GCaMP6 protein bound to calcium ions. In the case of the previous screening method, the therapeutic effect can be inferred through the overall degree of relaxation of dystonia symptoms, while the method used in the present invention can more effectively and in real time quantify the effect of the drug through the experimental animal. The present inventors performed real-time fluorescence analysis to analyze the correlation between dystonia symptoms and calcium ion concentration in nerve tissues in the topical transduction tottering mice prepared in Example 2. Particularly, to stimulate brain tissue through a polarization-maintaining single-mode optical fiber, a pulse laser of 488 nm wavelength with a frequency of 20 MHz was irradiated (left side in FIG. 4A). After the pulse laser irradiation, 509 nm wavelength fluorescence was measured using a TCSPC-based optical measurement system (Becker & Hickl, Germany). The fluorescence change rate (ΔF/F) was calculated based on the measured fluorescence. The ΔF/F was calculated by 100 × (F-Fₘₑₐₙ)/Fₘₑₐₙ, where Fₘₑₐₙ refers to the average value of the fluorescence intensity in the whole obtained fragment (Cui et al., Nature, 494(7436): 238- 242, 2013; Matthews et al., Bone, 84: 69-77, 2016). GCaMP6m fluorescence was recorded for 30 minutes when the mouse was in the open field box in the cage.

As shown in FIG. 4D, it was confirmed that the fluorescence change was not observed when the experimental animals were in the home, but the fluorescence signal was significantly increased when they were moved to the open field box and exposed to a stress environment. However, as shown in FIG. 4E, this increase in fluorescence signal was reduced to the level when staying at the home in the group administered with the selective 5-HT_{2A} antagonist MDL100907.

In addition, as shown in FIG. 4C, it was confirmed that the change in the signal of fluorescence is synchronized with the muscle attack due to dystonia. This suggests that it is possible to verify therapeutic efficacy of a drug candidate that can alleviate the symptoms of dystonia by measuring the calcium ion concentration in the cerebellum.

In addition, as shown in FIG. 4F, as the time exposed to the stress environment increases, the concentration of the calcium ion increases significantly in the control group, while no increase of the concentration of calcium ions in the cerebellum was observed in the group administered with the selective 5-HT_{2A} antagonist MDL100907, even when exposed to the stress environment for a long time.

On the other hand, as shown in FIG. 4B, in order to confirm motor activity and neuron activation in the cerebellum, the present inventors measured the concentration of calcium ions according to the behavioral state. As a result, it was confirmed that the concentration of calcium ions was particularly increased when the model animals showed myotonic posture compared when there was no movement or they walked.

These results can predict the relationship between stress and calcium activity in the cerebellum. In addition, the present inventors confirmed that the increase of the concentration of calcium ions due to stress in the dystonia animal model can be blocked trough 5-HT_{2A} antagonist from these findings.

### Experimental Example 4: Fluorescence analysis through confocal imaging

After anesthetizing tottering mice prepared in Example 2 and exposed to a stressed environment, first reperfusion with heparin sodium salt dissolved in PBS, followed by reperfusion with 4% formaldehyde dissolved in PBS. After the brains were excised, the extracted brains were fixed overnight with a 4% formaldehyde solution. After fixation, the brains were sliced to a thickness of 40 µm on a vibrating microtome (Leica, Germany). Images for the brains were obtained using a LSM 780 confocal microscope (Zeiss, Germany) and analyzed with ZEN 2009 imaging software (Zeiss, Germany).

As a result, green fluorescence by EGFP was confirmed in the fastigial nucleus, as shown in the right upper panel of FIG. 4A. This indicates that the GCaMP6 gene locally transduced into the cerebellum according to one embodiment of the present invention was normally expressed.

The present invention has been described with reference to the above-described examples, but these are merely exemplary, and those skilled in the art will understand that various modifications and equivalent other embodiments are possible therefrom. Therefore, the true technical scope of the present invention should be determined by the technical spirit of the appended claims.

### Industrial availability

The present invention can be used in the manufacture of a medicament capable of effectively alleviating dystonia symptoms caused by genetic or environmental factors, as well as dystonia symptoms that cause side effects of depression treatments.

## Claims

1. A pharmaceutical composition for treating dystonia or relieving pain caused by myotonic condition comprising the serotonin receptor 5-HT_{2A} inhibitor as an active ingredient.

2. The pharmaceutical composition according to claim 1, wherein the myotonic condition is caused by dystonia, cerebral palsy, myotonic dystrophy or spinal cord myopia.

3. The pharmaceutical composition according to claim 1, wherein the 5-HT_{2A} inhibitor is a 5-HT_{2A} antagonist or 5-HT_{2A} inverse agonist.

4. The pharmaceutical composition according to claim 1, wherein the 5-HT_{2A} antagonist is an antagonizing antibody specifically binding to the 5-HT_{2A} or a functional fragment of the antagonizing antibody, clozapine, olanzapin, qutiapine, risperidone, ziprasidone, aripiprazole, acenapine, amitriptyline, clomipramine, amitriptyline, clomipramine, cyproheptadine, eplivanserin, etoperidone, haloperidol, hydroxyzine, iloperidone, ketanserin, metyrseride, Mianserin, mirtazapine, nefazodone, pimavanserin, pizotifen, ritanserin, trazodone, or yohimbine.

5. The pharmaceutical composition according to claim 3, wherein the 5-HT_{2A} antagonist is a selective 5-HT_{2A} antagonist or 5-HT_{2A/2C} dual antagonist that does not act on other types of serotonin receptors.

6. The pharmaceutical composition according to claim 5, wherein the 5-HT_{2A} selective antagonist is eplivanserin, 2-alkyl-4-aryl-tetrahydro-pyramido-azepine, AMDA (9 -aminomethyl-9,10-dihydroanthracene), hydroxyzine, pizotifen, 5-methoxy-N-(4-bromobenzyl) tryptamine (5-MeO-NBpBrT), glemanserin, niaprazine, pimavanserin, volinanserin, or LY-367265.

7. The pharmaceutical composition according to calim 5, wherein the 5-HT_{2A/2C} dual antagonist is ritanserin, ketanserin, cyproheptadine, AC-90179, trazodone or etoperidone.

8. The pharmaceutical composition according to claim 4, wherein the functional fragment of the antagonizing antibody is Fab, Fab', F(ab')₂, scFv, diabody, tribody, sdAb, V_{H}H, nanobody, monobody, variable lymphocyte receptor (VLR), Affilin, Affimer, Affitin, Avimer, DARPin, Fynomer, or Affibody.

9. The pharmaceutical composition according to claim 3, wherein the 5-HT_{2A} inverse agonist is AC-90179, pimavanserin, nelotanserin, volinanserin or eplivanserin.

10. The pharmaceutical composition according to claim 1, wherein the myotonic is caused by excessive stress, an abnormal increase in serotonin following administration of a selective serotonin reuptake inhibitor or abnormal activation of the serotonin cycle.

11. The pharmaceutical composition according to claim 10, wherein the selective serotonin reuptake inhibitors citalopram, dapoxetine, escitalopram, fluvoxamine, paroxetine, fluoxetine, sultraline, zimeldin, or vortioxetine.

12. A method of screening candidate of therapeutic substance for treating dystonia or relieving pain caused by dystonia, comprising:
observing whether a series of test compounds or natural products inhibits serotonin receptor 5-HT_{2A}; and
selecting test compounds or natural products identified as inhibiting the 5-HT_{2A}.

13. The method according to claim 12, wherein further comprising:
confirming whether the test compounds or natural products confirmed to inhibit 5-HT_{2A} inhibits the function of the serotonin receptor except 5-HT_{2A}; and
selecting test compounds or natural products that does not inhibit the function of the serotonin receptor except the 5-HT_{2A}.

14. The method according to claim 12, wherein the observing whether a series of test compounds or natural products inhibits serotonin receptor 5-HT_{2A} is performed through various *in vitro, in vivo* or *in silico* analytical methods.

15. The method according to claim 14, wherein the in vitro analytical method is performed through reacting the serotonin receptor 5-HT_{2A}, a ligand thereof (e.g., serotonin) and the test compounds or natural products, and selecting test compounds or natural products that inhibit the binding of the serotonin 5-HT_{2A} and the ligand.

16. The method according to claim 14, the *in vitro* analytical method comprises:
analyzing the concentration or activity of the signal molecule downstream of 5-HT_{2A} after treating the test compounds or the natural products with cells expressing the serotonin receptor 5-HT_{2A}; and
selecting test compounds or natural products that inhibit the concentration or activity of the signal molecule downstreamin of 5-HT_{2A}.

17. The method according to claim 14, wherein the signal molecule is inositol triphosphate (IP₃), diacylglycerol (DAG), arachidonic acid (AA), 2-arachidonylglycerol (2-AG), Ca²⁺ or PKC.

18. The method according to claim 14, wherein the *in vivo* analytical method comprises:
inducing stress in the tottering animals having a mutation in P/Q type calcium channel by leaving the animals in a stress condition;
administering a test compound or a natural product to the tottering animals under stress; and
selecting a test compound or a natural product which significantly reduced the dystonia score of the tottering animals.

19. The method according to claim 14, wherein the *in vivo* analytical method comprises:
transducing a gene encoding calcium sensor protein topically into the cerebellum of a tottering animal having a mutation in P/Q type calcium channel in addition to the measurement of the dystonia score of the tottering animal;
inducing stress in the tottering animal by leaving the animal in a stress condition;
administering the test compound or natural product to the tottering animal under stress;
measuring the amount of calcium sensor protein bound to calcium in the cerebellum of the tottering animal; and
selecting a test compound or natural product that significantly lowers the amount of calcium sensor protein bound to calcium.

20. The method according to claim 19, wherein the calcium sensor protein is yellow camelon (YC), Inverse-Pericam, Camgroo, TN-L15, SynapCam or GCaMP.

21. The method according to claim 20, wherein the GCaMP is GCaMP1, GCamP2, GCaMP3, GCaMP4, GCaMP5 or GCaMP6.

22. Use of a serotonin receptor 5-HT_{2A} inhibitor in the manufacture of a pharmaceutical agent for treating dystonia or relieving pain caused by dystonia.
